# EUROPEAN PATENT SPECIFICATION

(11) **EP 4 307 984 B1**
(45) Date of publication and mention of the grant of the patent: **17.07.2024**
(21) Application number: 22708966.1
(22) Date of filing: 09.03.2022
(51) Int. Cl.: A61B 5/00, A61B 5/1455

(54) **A METHOD AND SYSTEM FOR ANALYSING OBJECTS**
VERFAHREN UND SYSTEM ZUR ANALYSE VON OBJEKTEN
PROCÉDÉ ET SYSTÈME D'ANALYSE D'OBJETS

(30) Priority: 18.03.2021 EP 21163502
(43) Date of publication of application: 24.01.2024
(73) Proprietor: Koninklijke Philips N.V., 5656 AG Eindhoven (NL)
(72) Inventor: PALERO, Jonathan Alambra, 5656 AG Eindhoven (NL); DAMODARAN, Mathivanan, 5656 AG Eindhoven (NL); BOURQUIN, Yannyk Parulian Julian, 5656 AG Eindhoven (NL); VERHAGEN, Rieko, 5656 AG Eindhoven (NL)
(74) Representative: Philips Intellectual Property & Standards
(86) International application number: PCT/EP2022/055959
(87) International publication number: WO 2022/194623

(56) References cited:
- US-A1- 2020 154 024
- US-A1- 2020 305 784
- FERIS ROGERIO ET AL: "Specular highlights detection and reduction with multi-flash photography", JOURNAL OF THE BRAZILIAN COMPUTER SOCIETY, 1 February 2006 (2006-02-01), Rio de Janeiro, pages 35 - 42, XP055929085, Retrieved from the Internet <URL:https://www.scielo.br/j/jbcos/a/nBCkb4KwBVPYkzM74B9nB6P/?lang=en&format=pdf> [retrieved on 20220608], DOI: 10.1007/BF03192386

## Description

### FIELD OF THE INVENTION

The invention relates to a method and system for analysing an object. Particularly although not exclusively, the method and system are suitable for analysing an object, such as human face or a skin surface, over time.

### BACKGROUND OF THE INVENTION

Analysis of an object typically involves analysis of images captured by a camera. However, the image may be distorted or comprise artifacts which make useful comparison between images, to monitor changes in an object over time, difficult.

Previously considered methods and systems for analysing objects are typically highly complex and confined to laboratory or clinical environments. Such systems comprise complex spectral cameras, and require strictly controlled ambient light conditions.

US 9,479,251 discloses a method for detecting modulated light comprising: receiving a set of images acquired by means of a rolling shutter camera having image acquisition settings; identifying in consecutive frames of said images-a pattern governed by the ratio between a modulation frequency, fc, of a modulated light source and the line rate, and-between consecutive frames a spatial shift of said pattern governed by the ratio between said modulation frequency fc, and said frame rate; and providing based on said pattern and spatial shift thereof, an estimate of the modulated light amplitude from said light source. Similar methods also using a rolling shutter camera are known from US 2020/154024 A1 and US 2020/305784 A1.

### SUMMARY OF THE INVENTION

A method according to the invention is defined in claim 1, and a system according to the invention is defined in claim 7. Further aspects of this disclosure are detailed below.

According to a first specific aspect, there is provided a method for analysing an object, the method comprising: illuminating the object with illumination provided by at least two illumination sources, each illumination source being defined by at least one distinct characteristic property; temporally modulating the illumination provided by each illumination source according to a modulation function; capturing a plurality of image frames representing the object using a rolling shutter image sensor; and demultiplexing the plurality of image frames into component images each representing the object under illumination from a respective illumination source, and an ambient image representing the object under an ambient light condition.

Each of the plurality of image frames may comprise a corresponding plurality of pixels, wherein each of the plurality of pixels may be defined by a pixel index corresponding to a location of the pixel within the image frames. The plurality of image frames may include at least three times as many image frames as there are illumination sources plus a further three image frames (i.e. if there are "n" illumination sources, then there are at least 3n+3 image frames). In other examples, the plurality of image frames may include at least three times as many image frames as there are illumination sources plus a further one image frame (i.e. if there are "n" illumination sources, then there are at least 3n+1 image frames), when is it assumed that the ambient light is constant or not modulating in time. In yet further examples, the plurality of image frames may include at least as many image frames as there are illumination sources plus a further one image frame (i.e. if there are "n" illumination sources, then there are at least n+1 image frames), when is it assumed that the ambient light is constant, or at least as many image frames as there are illumination sources plus a further three image frames, when the ambient light is modulated (i.e. if there are "n" illumination sources, then there are at least n+3 image frames).

In each of these examples, the modulation functions for each illumination source may be configured such that, for each of the plurality of image frames, the illumination of the object may be different when each corresponding pixel at the same pixel index is captured.

The modulation function for each illumination source may be a periodic function. Each periodic modulation function may have a frequency which is a non-integer multiple of a frame rate of the rolling shutter image sensor Each modulation function may have a phase, a waveform and a duty cycle, and the rolling shutter image sensor has a frame capture rate. The frequency of each modulation function may be greater than, and a non-integer multiple of, the frame capture rate.

The characteristic property of each illumination source may comprise a position relative to the rolling shutter image sensor and/or a wavelength.

The characteristic property of each illumination source may comprise a position relative to the image sensor and each component image may comprise a corresponding plurality of pixels, wherein each of the plurality of pixels has a pixel value and is defined by a pixel index corresponding to a location of the pixel within the component image. The method may further comprise generating a specular reflection-free image and/or a specular reflection-only image from the component images by computing, at each pixel index, a reflection-free pixel value based on a pixel value at a corresponding location within the component images.

The characteristic property of each illumination source may comprise a position relative to the image sensor and each component image and the ambient image comprises a corresponding plurality of pixels, wherein each of the plurality of pixels has a pixel value and is defined by a pixel index corresponding to a location of the pixel within the component images and within the ambient image respectively. The method may further comprise: for each pixel index, determining an x-component, a y-component and a z-component of a vector normal to a surface of the object at the pixel index; and generating a depth map from the component images and the ambient image by calculating, at each pixel index, a depth value based on the x-component, the y-component and the z-component.

Each component image and the ambient image may comprise a corresponding plurality of pixels, wherein each of the plurality of pixels has a pixel value and is defined by a pixel index corresponding to a location of the pixel within the component images and within the ambient image respectively. The method may further comprise generating an ambient light corrected image for each component image by subtracting, at each pixel index, the pixel value of the ambient image from the pixel value of the respective component image.

The object may comprise a skin surface, and the characteristic property of each illumination source may comprise a wavelength. The method may further comprise generating a physiological component map from the ambient light corrected image by determining, at each pixel index, a physiological parameter associated with the pixel value of the ambient light corrected image.

The physiological parameter may be selected from a group consisting of: an oxygenated haemoglobin level, a deoxygenated haemoglobin level and a melanin level.

The plurality of image frames may be a first plurality of image frames, the component images may be first component images, and the ambient image may be a first ambient image. The method may further comprise: capturing a second plurality of image frames representing the object using a roller shutter image sensor. The method may further comprise demultiplexing the second plurality of image frames into second component images and a second ambient image.

The ambient light corrected image may be a first ambient light corrected image. The method may further comprise: generating a second ambient light corrected image for each second component image. The method may further comprise generating a physiological component map from the second ambient light corrected image. The method may further comprise monitoring changes in the physiological component map between the first ambient light corrected image and the second ambient light corrected image.

At least one of the plurality of illumination sources may comprise a cluster of light sources. Each light source may be defined by a distinct wavelength. The method may further comprise demultiplexing the component images into light images each representing the object under illumination from a respective light source.

According to a second aspect, there is provided a non-transitory computer readable storage medium comprising computer-readable instructions that, when executed by a processor, causes the performance of the method in accordance with the first aspect.

According to a third aspect, there is provided a computer program that, when read by a computer, causes performance of the method in accordance with the first aspect.

According to a fourth aspect, there is provided a system for analysing objects, comprising: a plurality of illumination sources, wherein each illumination source is defined by at least one distinct characteristic property; a rolling shutter image sensor; and at least one processor; at least one memory comprising computer-readable instructions; wherein the at least one processor is configured to read the computer readable instructions and cause performance of a method in accordance with the first aspect.

These and other aspects will be apparent from and elucidated with reference to the embodiments described hereinafter.

### BRIEF DESCRIPTION OF THE DRAWINGS

Exemplary embodiments will now be described, by way of example only, with reference to the following drawings, in which:
Fig. 1 shows an example system for analysing an object;
Fig. 2a is a flowchart showing a first example method for analysing an object;
Fig. 2b shows an example plurality of image frames captured and demultiplexed according to the first example method of Fig. 2a;
Fig. 2c shows an example image frame captured together with a spatial illumination pattern;
Fig. 3a is a flowchart showing a second example method for analysing an object;
Fig. 3b shows an example plurality of image frames captured and processed to generate a specular reflection-free image according to the second example method of Fig. 3a;
Fig. 3c shows an example plurality of image frames captured and processed to generate a depth map generated according to the second example method shown of Fig. 3a; and
Fig. 4 is a flowchart showing a third example method for analysing an object.

### DETAILED DESCRIPTION OF THE EMBODIMENTS

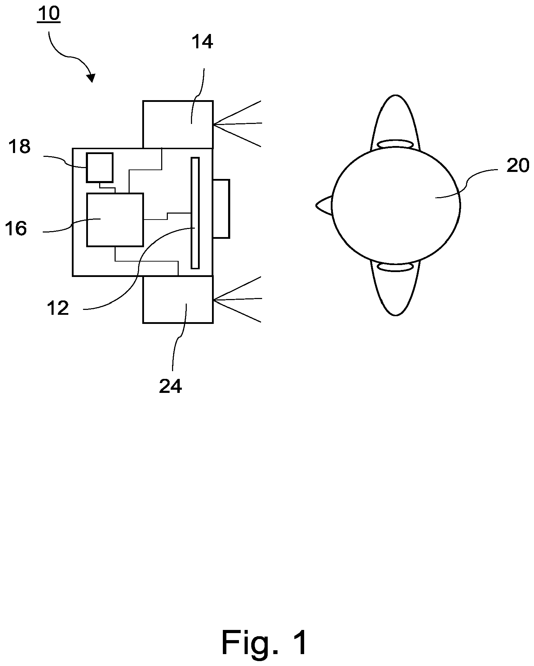

Fig. 1 shows a system 10 for analysing an object 20, the system 10 comprising a rolling shutter image sensor 12, a plurality of illumination sources 14, 24, a processor 16 and a memory 18 comprising computer readable instructions.

In this example, the plurality of illumination sources 14, 24 comprises a first illumination source 14 and a second illumination source 24. Each illumination source 14, 24 is defined by a distinct characteristic property. In this example, the characteristic property includes a position of the illumination sources 14, 24 relative to the rolling shutter image sensor 12, whereby the first illumination source 14 is located on a first side of the rolling shutter image sensor 12, and the second illumination source 24 is located on an opposing second side of the rolling shutter image sensor 12. In other examples, the characteristic property may additionally or alternatively comprise a wavelength of the illumination produced each illumination source.

The first illumination source 14 and the second illumination source 24 are each configured to illuminate the object 20. Each illumination source 14, 24 is connected to the processor 16, which is configured to temporally modulate the illumination provided by the first illumination source 14 and the second illumination source 24.

The rolling shutter image sensor 12 is connected to the processor 16 and is configured to capture image frames of an object 20, which in this example is a human face. The captured image frames are read and processed by the processor 16 and are stored in the memory 18.

The memory 18 comprises computer readable instructions and the processor 16 is configured to read the computer readable instructions to perform a method for analysing the object 20. The method for analysing the object 20 may be in accordance with any of the methods described herein.

In some examples, the system 10 may form part of or be incorporated into a personal smart device such as a smartphone or a household smart device such as a smart mirror.

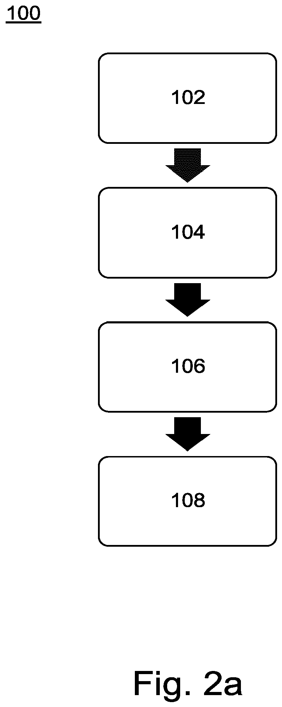

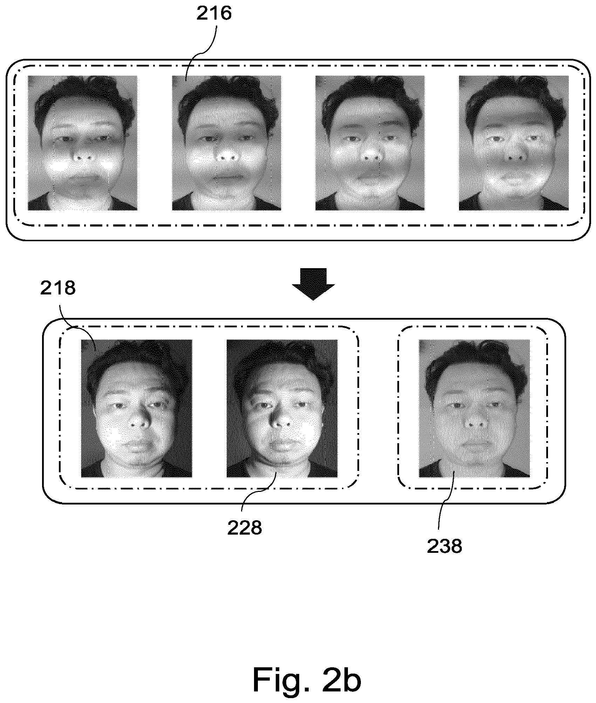

Fig. 2a is a flowchart showing a first example method 100 for analysing an object. In this example, the first example method 100 is performed by the system 10. In other examples, the first example method 100 may be performed by any suitable system. Fig. 2b shows the first example method 100 in terms of captured and processed image frames.

The first example method 100 commences at block 102, which comprises illuminating the object 20 with illumination provided by the at least two illumination sources 14, 24, each illumination source being defined by at least one distinct characteristic property.

At block 104, the first example method 100 comprises temporally modulating the illumination provided by each illumination source 14, 24 according to a respective modulation function. The modulation function modulates the amplitude of the illumination provided according to a predetermined function. In this example, the modulation function for each illumination source 14, 24 is a sinusoidal function having a frequency which is higher than a frame rate of the rolling shutter image sensor 12, and each illumination source 14, 24 is modulated out of phase. In other examples, the modulation function may be any periodic function, or may be a non-periodic function.

The first example method 100 also comprises block 106, comprising capturing a plurality of image frames 216 representing the object 20 using a rolling shutter image sensor 12. It can be seen from Fig. 2b that four image frames 216 are captured, and due to the modulation of the illumination sources 14, 24 while the rolling shutter image sensor 12 captures each image frame 216, different parts of the object 20 within a single image frame 216 are illuminated differently.

Each of the four example image frames 216 shown in Fig. 2b represent the object 20 under illumination from both the first illumination source 14 and the second illumination source 24. The image frames 216 captured by the rolling shutter image sensor 12 comprise a plurality of angled dark and light bands, which are part of a spatial light intensity pattern across each image frame 216. This spatial light intensity pattern is captured due to the use of the rolling shutter image sensor 12. The spatial light intensity pattern comprises a plurality of spatial intensity functions. Each spatial intensity function corresponds to the modulation function of a respective illumination source 14, 24, and as such is related to the illumination provided by a respective illumination source 14, 24.

At block 108, the first example method 100 continues by demultiplexing the plurality of image frames 216 into component images 218, 228 and an ambient image 238. Each component image 218, 228 represents the object 20 under illumination from a respective illumination source 14, 24. Therefore, a first component image 218 in this example represents the object 20 under illumination from the first illumination source 14, and a second component image 228 represents the object 20 under illumination from the second illumination source 24. The ambient image 238 represents the object 20 under an ambient light condition, when both illumination sources 14, 24 are off, or not illuminating the object 20. The illumination of the object 20 at each pixel from the first illumination source 14 (i.e. the amplitude of light received at the pixel from the first illumination source) is a first unknown variable, the illumination of the object 20 at each pixel from the second illumination source 24 (i.e. the amplitude of light received at the pixel from the second illumination source 24) is a second unknown variable, and the ambient illumination of the object 20 at each pixel (i.e. the amplitude of the light received at the pixel from the ambient light) is a third unknown variable. Therefore, provided that the modulation functions of the first illumination source 14 and the second illumination source 24 are known, and that the rolling shutter image sensor 12 is synchronised to the modulations functions (i.e. the rolling shutter image sensor 12 has a well-defined and constant frame rate, such that the timing of capturing each image frame 206 can be precisely mapped against the timing of the controlled modulation of the light sources 14, 24), and if it is assumed that the ambient light intensity does not change significantly over time then, if there are at least three image frames 216, the three unknown variables can be determined. In other words, each illumination source introduces one unknown, and the ambient light has one unknown, such that there needs to be n+1 image frames to resolve all of the unknowns, where n is the number of illumination sources.

In some examples, the rolling shutter image sensor may not have a constant frame rate, so although it has a known average frame rate, the actual start and finish time of capturing each image frame are unknown, such that timing of capturing each image frame cannot be precisely mapped against the modulation of the illumination sources. In such examples, the modulation frequency and phase of each illumination is also unknown, such that for each illumination source, all of the modulation frequency, phase and the amplitude are unknown, and the amplitude of the ambient light is also unknown, assuming that the ambient light is constant. Therefore, there are 3n+1 unknowns, where n is the number of illumination sources, such that there need to be 3n+1 image frames captured to resolve the unknown variables. In further examples, the ambient light may not be constant (i.e. it may also be modulated), thereby having an unknown modulation frequency and phase, such that this introduces a further two unknown variables, thereby requiring a further two image frames to resolve.

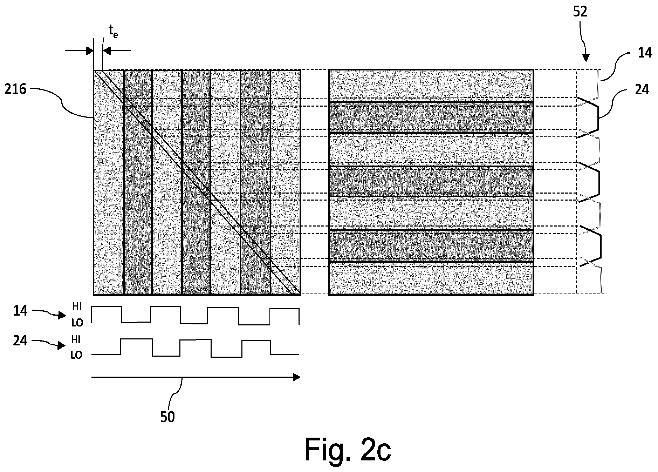

Fig. 2c shows an example image frame 216 and the illumination of the image frame 216 over time 50 by the two different illumination sources 14, 24. In this example, the illumination sources 14, 24 are modulated to alternately pulse, and in this example, there are 7 pulses in a single frame. The frequency of the light pulses may be more than the frequency of the frame capture rate of the rolling shutter image sensor 12.

Due to the nature of the rolling shutter image sensor 12 a band of the image sensor 12 is sensitive to light at any one time, with the size of the band dependent on the exposure time tₑ for each line of the rolling shutter image sensor 12. Each line of the rolling shutter image sensor 12 therefore captures light intensity with a spatial intensity function 52 corresponding to each illumination source 14, 24, thereby generating the spatial intensity pattern, as shown in Fig. 2c. Having a binary modulation of the illumination sources 14, 24 means that there is a predictable and easy to calculate spatial intensity function 52, even at the location in the image frame 216 which is exposed temporarily to illumination from more than one illumination source 14, 24. Minimising the exposure time minimises the size of the band which is sensitive at any one time, and therefore minimises the mixing of light from different illumination sources in any parts of the image frame 216, thereby requiring less processing power to demultiplex in block 108 of the first example method 100.

In order to ensure that every part of an image corresponding to an object is exposed to illumination from one illumination source 14, 24 over time, if the modulation of the illumination source is periodic, then it is necessary for each illumination source 14, 24 to have a frequency which is a non-integer multiple of the frame rate of the rolling shutter image sensor 12. This ensures that the spatial intensity function 52 for each illumination source 14, 24 drifts down the image over multiple image frames 216.

In one example, if the modulation function of each illumination source 14, 24 and the frame capture rate of the rolling shutter image sensor 12 is known, the corresponding spatial intensity function may be deduced. The spatial intensity function can then be used to generate a binary image mask for each of the captured image frames 216 corresponding to each illumination source and to the object under an ambient light condition. The binary image masks may be used to remove pixels in each image frame 216 which are not relevant to the respective illumination source 14, 24. In other words, the binary image mask for an illumination source is used to remove pixels which were not sensitive at a time when the respective illumination source was illuminating the object 20. The binary image masks for each of the captured images corresponding to the first illumination source 14 are then used to construct a first example component image 218 representing the object 20 under illumination from the first illumination source 14 only.

The binary image masks for each of the captured image frames 216 corresponding to the second illumination source 24 are then used to construct a second example component image 228 representing the object 20 under illumination from the second illumination source 24 only. In a similar way, the binary image masks for each of the captured images corresponding to the object 20 under an ambient light condition are then used to construct the ambient image 238 representing the object 20 under ambient light conditions only.

The modulation function for each illumination source 14, 24 having a periodic function in this example simplifies the demultiplexing in block 108 because the spatial intensity function can be derived easily from this, thereby requiring less processing power. It will be appreciated that in some examples, the modulation function may not be periodic, or may have a frequency lower than or equal to the frame rate of the rolling shutter image sensor. In some examples, the modulation function may be a binary function where the illumination source is either on or off, such as being pulsed, or continuous such as the amplitude being modulated in a sine wave.

Each illumination source need not have the same modulation function. The modulation functions of the plurality of illumination sources may be different, or if they are the same, may be out of phase with one another.

The modulation function may have a phase, a frequency, a waveform and a duty cycle.

The waveform may be a geometric waveform. The geometric waveform may be a trigonometric waveform; a hyperbolic waveform, a trapezoidal waveform or a square waveform, for example. The waveform may comprise a plurality of superimposed waveforms. If the waveform is a geometric waveform, the demultiplexing operation carried out at block 108 may require less processing power, since the spatial intensity function will be simple to calculate.

If the object is a human face, as described with reference to the example of Fig. 1, it is desirable to ensure that a human observer does not perceive flickering in the plurality of illumination sources. Therefore, the frequency of each modulation function may be greater than or equal to 60 Hz. In some examples, the frequency of each modulation function may be greater than or equal to 200 Hz.

Depending on the characteristic property of each illumination source and an ambient operating condition, a frequency of each modulation function at which a human observer will not perceive any flickering in the plurality of illumination sources may be approximately 60 Hz. Irrespective of the characteristic property of each illumination source and the ambient operating condition, a human observer will not perceive any flickering in the plurality of illumination sources when the frequency of each modulation function is greater than or equal to 200 Hz.

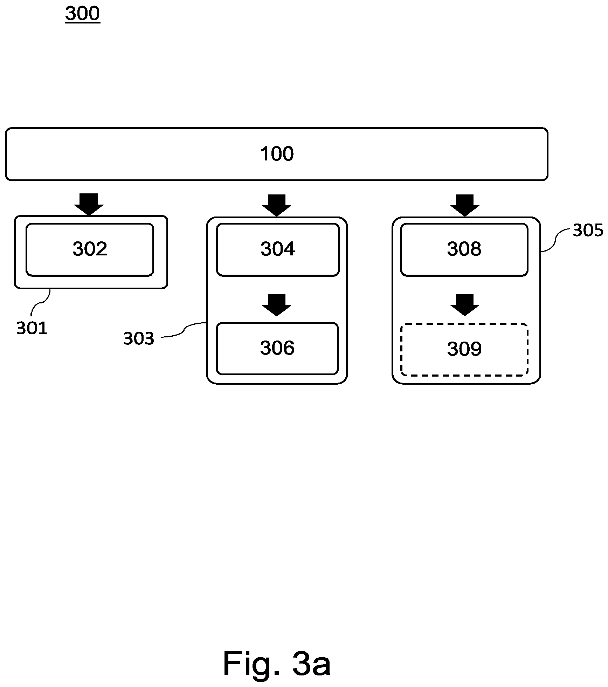

Fig. 3a is a flowchart showing a second example method 300 for analysing an object 20. The method comprises a plurality of branches of sub-methods to analyse the objects 20.

The second example method 300 commences by performing the first example method 100 as shown in Fig. 2a, comprising blocks 102, 104, 106 and 108 as previously described. The output of the first example method 100 is the component images 218, 228 corresponding to the object 20 illuminated by each illumination source 14, 24 respectively, and the ambient image 238. From the first example method 100, the second example method 300 may proceed to any one of a first sub-method 301, a second sub-method 303 and a third sub-method 305. The second example method 300 may include each one of these sub-methods or any combination of them.

Each component image 218, 228 and ambient image 238 demultiplexed from the plurality of image frames 216 in block 108 comprises a corresponding plurality of pixels, wherein each of the plurality of pixels has a pixel value and is defined by a pixel index corresponding to a location of the pixel within the respective component image 218, 228 or ambient image 238.

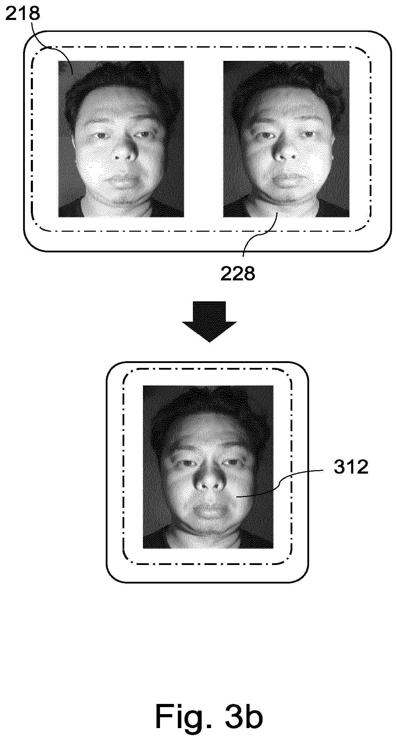

The first sub-method 301 comprises block 302, illustrated by Fig. 3b which shows steps of the first sub-method 301 in terms of the image frames which are captured and processed. The second example method 300 may proceed to the first sub-method 301 provided that the characteristic property of each illumination source 14, 24 comprises a position relative to the rolling shutter image sensor 12.

Block 302 comprises generating a specular reflection-free image 312 from the component images 218, 228 by computing, at each pixel index, a reflection-free pixel value based on a pixel value at a corresponding location within the component images 218, 228. For example, the specular reflection-free image 312 may be generated by selecting, at each pixel index, the lowest pixel value from the component images 218, 228 at a corresponding location within the component images 218, 228, such as at the same pixel index.

Block 302 may additionally or alternatively comprise generating a specular reflection-only image from the component images 218, 228 by computing, at each pixel index, a reflection-only pixel value based on a pixel value at a corresponding location within the component images 218, 228. For example, the specular reflection-only image may be generated by selecting, at each pixel index, the highest pixel value from the component images 218, 228 at a corresponding location within the component images 218, 228, such as at the same pixel index

In another example, the reflection-free pixel value at each pixel index is computed by determining a local light intensity gradient from the component images at a corresponding location within the component images. The local light intensity gradient may then be used to determine a contribution of specular reflection at a corresponding location within each component image, which may then be eliminated to compute the reflection-free pixel value. This is described in more detail in Feris, R., Raskar, R., Tan, KH. et al. Specular highlights detection and reduction with multi-flash photography. J Braz Comp Soc 12, 35-42 (2006).

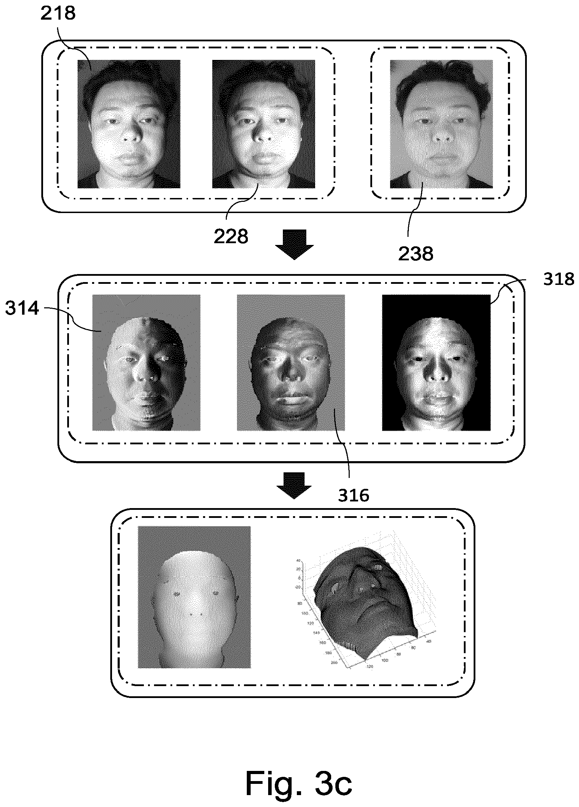

The second example method 300 may proceed from the first example method 100 to second sub-method 303. The second example method 300 with the second sub-method 303 is illustrated by Fig. 3c which shows steps of the method in terms of image frames captured and processed. The second example method 300 may proceed to second sub-method 303 provided that the characteristic property of each illumination source 14, 24 comprises a position relative to the rolling shutter image sensor 12.

The second sub-method 303 begins with block 304 which comprises determining, for each pixel index, an x-component, a y-component and a z-component of a vector normal to a surface of the object 20 at the pixel index based on the component images 218, 228 and the ambient image 238. Fig. 3c shows a first normal vector map (comprising an x-, y-, and z-component map) 314 derived from the first component image 218, a second normal vector map 316 derived from the second component image 228 and a third normal vector map 318 derived from the ambient image 238.

The second sub-method 303 then proceeds to block 306, comprising generating a depth map from the component images 218, 228 and the ambient image 238 by calculating, at each pixel index, a depth value based on the x-component, the y-component and the z-component.

Second sub-method 303 may be carried out, for example, by using a Lambertian reflectance model such as described in Hernández, C, Vogiatzis, G & Cipolla, R 2008, 'Multiview photometric stereo', IEEE Transactions on Pattern Analysis and Machine Intelligence, vol. 30, no. 3, pp. 548-554.

The second example method 300 may proceed from the first example method 100 to the third sub-method 305 which begins with block 308. Block 308 comprises generating an ambient light corrected image for each component image by subtracting, at each pixel index, the pixel value of the ambient image 238 from the pixel value of the respective component image 218, 228.

Each component image 218, 228 represents the object under illumination from a respective illumination source 14, 24, and so the respective ambient light corrected image represents the object 20 under illumination from a respective illumination source 14, 24 having corrected for an influence of an ambient light condition.

In this example, following block 308, the third sub-method 305 proceeds to block 309. In other examples, the third sub-method 305 may end with block 308 and not proceed to block 309. The third sub-method 305 may proceed to block 309 provided that the characteristic property of each illumination source comprises a wavelength. The component images 218, 228 which are generated by the first example method 100 therefore each represent the object 20 illuminated by a different wavelength of light, and each ambient light corrected image represents the object 20 illuminated by a different wavelength of light which has been corrected for an ambient light condition.

Block 309 comprises generating a physiological component map from the ambient light corrected images by determining, at each pixel index, a physiological parameter associated with the pixel value of the ambient light corrected image. The physiological parameter may be oxygenated haemoglobin level, a deoxygenated haemoglobin level or a melanin level, for example. The physiological parameter may provide an indication of a health status of the object 20 if the object forms part of a biological organism, such as when the object comprises a skin surface.

In some examples, generating a physiological component map may be based on a Kubelka-Munk model, such as described in Setiadi, Iwan & Nasution, Aulia & Chandra, Theodore. (2019). A new LED-based multispectral imaging system for blood and melanin content estimation: The validation. AIP Conference Proceedings 2193.050017.10.1063/1.5139390.

Any algorithm used at block 309 assumes that the physiological parameter is associated with the pixel value of a respective ambient light corrected image, which in turn represents the object under illumination from a respective illumination source.

In other examples, a numerical model may be used to determine, at each pixel index, a physiological parameter associated with the pixel value of the ambient light corrected image. The numerical model may be based on a machine-learning algorithm for predicting the physiological parameter based on the pixel value of the ambient-light corrected image.

It will be understood that blocks 302, 304, 306, 308 and 309 may be conducted in any suitable order.

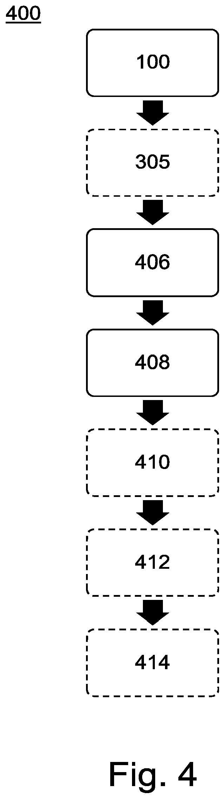

Fig. 4 is a flowchart showing a third example method 400 for analysing an object.

The third example method 400 commences by performing the first example method 100 as described with reference to Fig. 2a, comprising blocks 102, 104, 106 and 108. In the first example method, the plurality of image frames 216 captured is a first plurality of image frames 216, the component images 218, 228 are first component images 218, 228, and the ambient image 238 is a first ambient image 238. The third example method 400 proceeds to perform the third sub-method 305 including block 308 and block 309, as described with reference to Fig. 3a, in which a first ambient light corrected image is generated for each first component image 218, 228, and in which a physiological parameter map is generated. In other examples, third sub-method 305 may be omitted from the third example method 400, or block 309 may be omitted from the third sub-method 305 in the third example method 400.

The third example method 400 further comprises block 406, which comprises capturing a second plurality of images frames representing the object 20 using the rolling shutter image sensor 12. The second plurality of image frames are captured in a manner similar to block 102, described with reference to Fig. 2a.

The third example method 400 proceeds to block 408, comprising demultiplexing the second plurality of image frames into second component images each representing the object 20 under illumination from a respective illumination source 14, 24, and a second ambient image representing the object 20 under an ambient light condition. Block 408 may be performed in a manner similar to blocks 104-108 described with reference to Fig. 2a.

The third example method 400 then proceeds to block 410, in which a second ambient light corrected image is generated in a manner similar to block 308 in the third sub-method 305 described with reference to Fig. 3a.

The third example method 400 further proceeds to block 412, at which a physiological parameter map is generated in a similar manner to the physiological parameter map which is generated at block 309 in the third sub-method 305, described with reference to Fig. 3a. In some examples, if the third sub-method 305 is omitted from the third example method 400, then blocks 410-412 may also be omitted from the third example method. If block 309 is omitted from the third sub-method 305 in the third example method 400, then block 412 may also be omitted.

The third example method 400 proceeds to block 414, in which changes in the physiological component map between the first ambient light corrected image and the second ambient light corrected image are monitored.

Although the third example method 400 has been described as generating ambient light corrected images and physiological component maps to be monitored to detect changes over time, it will be appreciated that a similar method may be applied to depth maps described with reference to Fig. 3a and Fig. 3c, in which block 414 monitors changes in a depth map or specular reflection free images over time.

Although it has been described that there are two illumination sources in the examples, it will be appreciated that there can be any suitable number of illumination sources. In any of the previously described methods, at least one illumination source may comprise a cluster of light sources, wherein each light source is defined by a distinct wavelength. The method may then further comprise, at block 108, demultiplexing the component images into light images each representing the object under illumination from a respective light source.

In one example, if the component images are in an RGB format and a relative spectral sensitivity of each RGB channel of the rolling shutter image sensor is known, the contribution of the illumination provided by each light source to each component image may be determined. The contribution of the illumination provided by each light source may then be extracted from each component image to generate a light image representing the object 20 under illumination from each light source.

The wavelength of each light source may be chosen so that the cluster of light sources is perceived by a human observer as a selected colour, such as white light. This reduces the perception of flickering coloured lights to a user, so as to be less intrusive to a user.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method for analysing an object (20) comprising a skin surface, the method comprising:
illuminating the object (20) with illumination provided by at least two illumination sources (14, 24), each illumination source (14, 24) being defined by at least one distinct characteristic property, the at least one distinct characteristic property comprising a wavelength;
temporally modulating the illumination provided by each illumination source (14, 24) according to a modulation function, wherein the modulation function for each illumination source (14, 24) is a periodic function and wherein each periodic modulation function has a frequency which is a non-integer multiple of a frame rate of the rolling shutter image sensor (12);
capturing a first plurality of image frames (216) and a second plurality of image frames (216) representing the object (20) using a rolling shutter image sensor (12), the first and second plurality of image frames each including at least n+1 image frames, where n is the number of illumination sources;
wherein the rolling shutter image sensor has a well-defined and constant frame rate; and
demultiplexing the first plurality of image frames (216) and the second plurality of image frames (216) respectively into first and second component images (218, 228) each representing the object (20) under illumination from a respective illumination source (14, 24), and a first and second ambient image (238) representing the object (20) under an ambient light condition;
wherein the first and second component images and the first and second ambient images are constructed via a binary image mask, the binary image mask being generated using a spatial intensity function deduced from the modulation function of each illumination source (14,20) and the frame rate of the rolling shutter image sensor (20);
wherein each component image (218, 228) and ambient image (238) comprises a corresponding plurality of pixels, wherein each of the plurality of pixels has a pixel value and is defined by a pixel index corresponding to a location of the pixel within the component images (218, 228) and within the ambient images (238) respectively;
generating a first ambient light corrected image for each first component image (218, 228) and a second ambient light corrected image for each second component image (218, 228) by subtracting, at each pixel index, the pixel value of the respective ambient image (238) from the pixel value of the respective component image (218, 228); and
generating a physiological component map from the first ambient light corrected image and from the second ambient light corrected image by determining, at each pixel index, a physiological parameter associated with the pixel value of the ambient light corrected image; and
monitoring changes in the physiological component map between the first ambient light corrected image and the second ambient light corrected image;
wherein the physiological parameter is selected from a group consisting of an oxygenated haemoglobin level, a deoxygenated haemoglobin level and a melanin level.

2. The method according to claim 1, wherein each modulation function has a phase, a waveform and a duty cycle, and the rolling shutter image sensor (12) has a frame capture rate; and
wherein the frequency of each modulation function is greater than, and a non-integer multiple of, the frame capture rate.

3. The method according to any preceding claim, wherein the characteristic property of each illumination source (14, 24) comprises a position relative to the rolling shutter image sensor (12) and/or a wavelength.

4. The method according to any preceding claim, wherein
the characteristic property of each illumination source (14, 24) comprises a position relative to the image sensor;
each component image (218, 228) comprises a corresponding plurality of pixels, wherein each of the plurality of pixels has a pixel value and is defined by a pixel index corresponding to a location of the pixel within the component image (218, 228);
the method further comprising:
generating a specular reflection-free image (312) and/or a specular reflection-only image from the component images (218, 228) by computing, at each pixel index, a reflection-free pixel value based on a pixel value at a corresponding location within the component images (218, 228).

5. The method according to any preceding claim, wherein:
the characteristic property of each illumination source (14, 24) comprises a position relative to the image sensor;
each component image (218, 228) and the ambient image (238) comprises a corresponding plurality of pixels, wherein each of the plurality of pixels has a pixel value and is defined by a pixel index corresponding to a location of the pixel within the component images (218, 228) and within the ambient image (238) respectively;
the method further comprising:
for each pixel index, determining an x-component, a y-component and a z-component of a vector normal to a surface of the object (20) at the pixel index;
generating a depth map from the component images (218, 228) and the ambient image (238) by calculating, at each pixel index, a depth value based on the x-component, the y-component and the z-component.

6. The method according to any to any preceding claim, wherein at least one of the plurality of illumination sources (14, 24) comprises a cluster of light sources, wherein each light source is defined by a distinct wavelength, and wherein the method further comprises demultiplexing the component images (218, 228) into light images each representing the object (20) under illumination from a respective light source.

7. A system (10) for analysing objects (20), comprising:
a plurality of illumination sources (14, 24), wherein each illumination source (14, 24) is defined by at least one distinct characteristic property;
a rolling shutter image sensor (12); and
at least one processor (16);
at least one memory (18) comprising computer-readable instructions;
wherein the at least one processor (16) is configured to read the computer readable instructions and cause performance of a method in accordance with any of claims 1-6.

## Patentansprüche

1. Verfahren zum Analysieren eines Objekts (20), das eine Hautoberfläche umfasst, wobei das Verfahren umfasst:
Beleuchten des Objekts (20) mit Beleuchtung, die von mindestens zwei Beleuchtungsquellen (14, 24) bereitgestellt wird, wobei jede Beleuchtungsquelle (14, 24) durch mindestens eine eindeutige charakteristische Eigenschaft definiert ist, wobei die mindestens eine eindeutige charakteristische Eigenschaft eine Wellenlänge umfasst;
zeitliches Modulieren der von jeder Beleuchtungsquelle (14, 24) bereitgestellten Beleuchtung gemäß einer Modulationsfunktion, wobei die Modulationsfunktion für jede Beleuchtungsquelle (14, 24) eine periodische Funktion ist, und wobei jede periodische Modulationsfunktion eine Frequenz aufweist, die ein nicht ganzzahliges Vielfaches einer Bildrate des Rolling-Shutter-Bildsensors (12) ist;
Erfassen einer ersten Vielzahl von Einzelbildern (216) und einer zweiten Vielzahl von Einzelbildern (216), die das Objekt (20) darstellen, unter Verwendung eines Rolling-Shutter-Bildsensors (12), wobei die erste und die zweite Vielzahl von Einzelbildern jeweils mindestens n+1 Einzelbilder beinhalten, wobei n die Anzahl von Beleuchtungsquellen ist;
wobei der Rolling-Shutter-Bildsensor eine wohldefinierte und konstante Bildrate aufweist; und
Demultiplexen der ersten Vielzahl von Einzelbildern (216) und der zweiten Vielzahl von Einzelbildern (216) in ein erstes und ein zweites Komponentenbild (218, 228), die jeweils das Objekt (20) unter Beleuchtung durch eine jeweilige Beleuchtungsquelle (14, 24) darstellen, und ein erstes und ein zweites Umgebungsbild (238), die das Objekt (20) unter Umgebungslichtbedingung darstellen;
wobei das erste und das zweite Komponentenbild und das erste und das zweite Umgebungsbild über eine binäre Bildmaske aufgebaut werden, wobei die binäre Bildmaske unter Verwendung einer räumlichen Intensitätsfunktion erzeugt wird, die aus der Modulationsfunktion jeder Beleuchtungsquelle (14, 20) und der Bildrate des Rolling-Shutter-Bildsensors (20) abgeleitet wird;
wobei jedes Komponentenbild (218, 228) und Umgebungsbild (238) eine entsprechende Vielzahl von Pixeln umfasst, wobei jedes der Vielzahl von Pixeln einen Pixelwert aufweist und durch einen Pixelindex definiert ist, der einer Position des Pixels innerhalb der Komponentenbilder (218, 228) bzw. innerhalb der Umgebungsbilder (238) entspricht;
Erzeugen eines ersten umgebungslichtkorrigierten Bildes für jedes erste Komponentenbild (218, 228) und eines zweiten umgebungslichtkorrigierten Bildes für jedes zweite Komponentenbild (218, 228) durch Subtrahieren, an jedem Pixelindex, des Pixelwerts des jeweiligen Umgebungsbildes (238) vom Pixelwert des jeweiligen Komponentenbildes (218, 228); und
Erzeugen einer physiologischen Komponentenkarte aus dem ersten umgebungslichtkorrigierten Bild und aus dem zweiten umgebungslichtkorrigierten Bild durch Bestimmen, an jedem Pixelindex, eines physiologischen Parameters, der dem Pixelwert des umgebungslichtkorrigierten Bilds zugeordnet ist; und
Überwachen von Änderungen der physiologischen Komponentenkarte zwischen dem ersten umgebungslichtkorrigierten Bild und dem zweiten umgebungslichtkorrigierten Bild;
wobei der physiologische Parameter ausgewählt ist aus einer Gruppe, bestehend aus: einem Pegel an oxygeniertem Hämoglobin, einem Pegel an deoxygeniertem Hämoglobin und einem Melaninpegel.

2. Verfahren nach Anspruch 1, wobei jede Modulationsfunktion eine Phase, eine Wellenform und einen Tastgrad aufweist, und der Rolling-Shutter-Bildsensor (12) eine Bilderfassungsrate aufweist; und
wobei die Frequenz jeder Modulationsfunktion größer als die Bilderfassungsrate und ein nicht ganzzahliges Vielfaches davon ist.

3. Verfahren nach einem vorstehenden Anspruch, wobei die charakteristische Eigenschaft jeder Beleuchtungsquelle (14, 24) eine Position relativ zum Rolling-Shutter-Bildsensor (12) und/oder eine Wellenlänge umfasst.

4. Verfahren nach einem vorstehenden Anspruch, wobei
die charakteristische Eigenschaft jeder Beleuchtungsquelle (14, 24) eine Position relativ zum Bildsensor umfasst;
jedes Komponentenbild (218, 228) eine entsprechende Vielzahl von Pixeln umfasst, wobei jedes der Vielzahl von Pixeln einen Pixelwert aufweist und durch einen Pixelindex definiert ist, der einer Position des Pixels innerhalb des Komponentenbildes (218, 228) entspricht;
wobei das Verfahren weiter umfasst:
Erzeugen eines spiegelreflexionsfreien Bildes (312) und/oder eines Nur-Spiegelreflexionsbildes aus den Komponentenbildern (218, 228) durch Berechnen, an jedem Pixelindex, eines reflexionsfreien Pixelwerts auf Basis eines Pixelwerts an einer entsprechenden Position innerhalb der Komponentenbilder (218, 228).

5. Verfahren nach einem vorstehenden Anspruch, wobei:
die charakteristische Eigenschaft jeder Beleuchtungsquelle (14, 24) eine Position relativ zum Bildsensor umfasst;
jedes Komponentenbild (218, 228) und das Umgebungsbild (238) eine entsprechende Vielzahl von Pixeln umfasst, wobei jedes der Vielzahl von Pixeln einen Pixelwert aufweist und durch einen Pixelindex definiert ist, der einer Position des Pixels innerhalb der Komponentenbilder (218, 228) bzw. innerhalb des Umgebungsbildes (238) entspricht;
wobei das Verfahren weiter umfasst:
Bestimmen, für jeden Pixelindex, einer x-Komponente, einer y-Komponente und einer z-Komponente eines Vektors, der normal zu einer Oberfläche des Objekts (20) am Pixelindex ist;
Erzeugen einer Tiefenkarte aus den Komponentenbildern (218, 228) und dem Umgebungsbild (238) durch Berechnen, an jedem Pixelindex, eines Tiefenwerts auf Basis der x-Komponente, der y-Komponente und der z-Komponente.

6. Verfahren nach einem vorstehenden Anspruch, wobei mindestens eine der Vielzahl von Beleuchtungsquellen (14, 24) eine Gruppe von Lichtquellen umfasst, wobei jede Lichtquelle durch eine eindeutige Wellenlänge definiert ist, und wobei das Verfahren weiter das Demultiplexen der Komponentenbilder (218, 228) in Lichtbilder umfasst, die jeweils das Objekt (20) unter Beleuchtung von einer jeweiligen Lichtquelle darstellen.

7. System (10) zum Analysieren von Objekten (20), umfassend:
eine Vielzahl von Beleuchtungsquellen (14, 24), wobei jede Beleuchtungsquelle (14, 24) durch mindestens eine eindeutige charakteristische Eigenschaft definiert ist;
einen Rolling-Shutter-Bildsensor (12); und
mindestens einen Prozessor (16);
mindestens einen Speicher (18), der computerlesbare Anweisungen umfasst;
wobei der mindestens eine Prozessor (16) so konfiguriert ist, dass er die computerlesbaren Anweisungen liest und Durchführung eines Verfahrens nach einem der Ansprüche 1-6 veranlasst.

## Revendications

1. Procédé d'analyse d'un objet (20) comprenant une surface cutanée, le procédé comprenant :
l'éclairage de l'objet (20) avec un éclairage fourni par au moins deux sources d'éclairage (14, 24), chaque source d'éclairage (14, 24) étant définie par au moins une propriété caractéristique distincte, la au moins une propriété caractéristique distincte comprenant une longueur d'onde ;
la modulation temporelle de l'éclairage fourni par chaque source d'éclairage (14, 24) selon une fonction de modulation, dans lequel la fonction de modulation pour chaque source d'éclairage (14, 24) est une fonction périodique et dans lequel chaque fonction de modulation périodique présente une fréquence qui est un multiple non entier d'une fréquence de trames du capteur d'image à volet roulant (12) ;
la capture d'une première pluralité de trames d'image (216) et d'une seconde pluralité de trames d'image (216) représentant l'objet (20) en utilisant un capteur d'image à volet roulant (12), les première et seconde pluralités de trames d'image incluant chacune au moins n+1 trames d'image, où n est le nombre de sources d'éclairage ;
dans lequel le capteur d'image à volet roulant présente une fréquence de trames bien définie et constante ; et
le démultiplexage de la première pluralité de trames d'image (216) et de la seconde pluralité de trames d'image (216) respectivement en première et seconde images composantes (218, 228) représentant chacune l'objet (20) sous l'éclairage d'une source d'éclairage (14, 24) respective, et en première et seconde images ambiantes (238) représentant l'objet (20) dans des conditions de lumière ambiante ;
dans lequel les première et seconde images composantes et les première et seconde images ambiantes sont construites par l'intermédiaire d'un masque d'image binaire, le masque d'image binaire étant généré en utilisant une fonction d'intensité spatiale déduite de la fonction de modulation de chaque source d'éclairage (14, 20) et de la fréquence de trames du capteur d'image à volet roulant (20) ;
dans lequel chaque image composante (218, 228) et image ambiante (238) comprennent une pluralité correspondante de pixels, dans lequel chacun de la pluralité de pixels présente une valeur de pixel et est défini par un indice de pixel correspondant à un emplacement du pixel dans les images composantes (218, 228) et dans les images ambiantes (238) respectivement ;
la génération d'une première image corrigée en lumière ambiante pour chaque première image composante (218, 228) et d'une seconde image corrigée en lumière ambiante pour chaque seconde image composante (218, 228) en soustrayant, à chaque indice de pixel, la valeur de pixel de l'image ambiante (238) respective de la valeur de pixel de l'image composante (218, 228) respective ; et
la génération d'une carte de composantes physiologiques à partir de la première image corrigée en lumière ambiante et à partir de la seconde image corrigée en lumière ambiante en déterminant, à chaque indice de pixel, un paramètre physiologique associé à la valeur de pixel de l'image corrigée en lumière ambiante ; et
la surveillance de changements dans la carte de composantes physiologiques entre la première image corrigée en lumière ambiante et la seconde image corrigée en lumière ambiante ;
dans lequel le paramètre physiologique est sélectionné dans un groupe consistant en : un taux d'hémoglobine oxygénée, un taux d'hémoglobine désoxygénée et un taux de mélanine.

2. Procédé selon la revendication 1, dans lequel chaque fonction de modulation présente une phase, une forme d'onde et un cycle de service, et le capteur d'image à volet roulant (12) présente une fréquence de capture de trame ; et
dans lequel la fréquence de chaque fonction de modulation est supérieure à la fréquence de capture de trame, et est un multiple non entier de celle-ci.

3. Procédé selon une quelconque revendication précédente, dans lequel la propriété caractéristique de chaque source d'éclairage (14, 24) comprend une position par rapport au capteur d'image à volet roulant (12) et/ou une longueur d'onde.

4. Procédé selon une quelconque revendication précédente, dans lequel
la propriété caractéristique de chaque source d'éclairage (14, 24) comprend une position par rapport au capteur d'image ;
chaque image composante (218, 228) comprend une pluralité correspondante de pixels, dans lequel chacun de la pluralité de pixels présente une valeur de pixel et est défini par un indice de pixel correspondant à un emplacement du pixel dans l'image composante (218, 228) ;
le procédé comprenant en outre :
la génération d'une image sans réflexion spéculaire (312) et/ou d'une image à réflexion spéculaire uniquement à partir des images composantes (218, 228) en calculant, à chaque indice de pixel, une valeur de pixel sans réflexion sur la base d'une valeur de pixel à un emplacement correspondant dans les images composantes (218, 228).

5. Procédé selon une quelconque revendication précédente, dans lequel :
la propriété caractéristique de chaque source d'éclairage (14, 24) comprend une position par rapport au capteur d'image ;
chaque image composante (218, 228) et l'image ambiante (238) comprennent une pluralité correspondante de pixels, dans lequel chacun de la pluralité de pixels présente une valeur de pixel et est défini par un indice de pixel correspondant à un emplacement du pixel dans les images composantes (218, 228) et dans l'image ambiante (238) respectivement ;
le procédé comprenant en outre :
pour chaque indice de pixel, la détermination d'une composante x, d'une composante y et d'une composante z d'un vecteur normal à une surface de l'objet (20) à l'indice de pixel ;
la génération d'une carte de profondeur à partir des images composantes (218, 228) et de l'image ambiante (238) en calculant, à chaque indice de pixel, une valeur de profondeur sur la base de la composante x, de la composante y et de la composante z.

6. Procédé selon une quelconque revendication précédente, dans lequel au moins l'une de la pluralité de sources d'éclairage (14, 24) comprend un groupe de sources de lumière, dans lequel chaque source de lumière est définie par une longueur d'onde distincte, et dans lequel le procédé comprend en outre le démultiplexage des images composantes (218, 228) en images lumineuses représentant chacune l'objet (20) sous l'éclairage d'une source de lumière respective.

7. Système (10) pour analyser des objets (20), comprenant :
une pluralité de sources d'éclairage (14, 24), dans lequel chaque source d'éclairage (14, 24) est définie par au moins une propriété caractéristique distincte ;
un capteur d'image à volet roulant (12) ; et
au moins un processeur (16) ;
au moins une mémoire (18) comprenant des instructions lisibles par ordinateur ;
dans lequel le au moins un processeur (16) est configuré pour lire les instructions lisibles par ordinateur et provoquer l'exécution d'un procédé conformément à l'une quelconque des revendications 1-6.
